# FASCICULE DE BREVET EUROPEEN

(11) **EP 2 532 587 B1**
(45) Date de publication et mention de la délivrance du brevet: **07.10.2015**
(21) Numéro de dépôt: 12003963.1
(22) Date de dépôt: 21.05.2012
(51) Int. Cl.: B64C 27/00, G01M 7/00, G01M 17/00

(54) **Procédé technique, dispositif de maintenance, et aéronef**
Technisches Verfahren und Vorrichtung zur Wartung, sowie Luftschiff
Technical method and device for maintenance, and aircraft

(30) Priorité: 07.06.2011 FR 1101725
(43) Date de publication de la demande: 12.12.2012
(73) Titulaire: Airbus Helicopters, 13725 Marignane Cedex (FR)
(72) Inventeur: Pire, Richard, F-13800 Istres (FR)
(74) Mandataire: GPI & Associés

(56) Documents cités:
- EP-A2- 0 541 277
- A Oldersma ET AL: "Airframe loads & usage monitoring of the CH-47D "Chinook" helicopter of the Royal Netherlands Air Force", , 3 juin 2011 (2011-06-03), XP055016804, Extrait de l'Internet: URL:http://www.nlr.nl/?id=17188&l=en [extrait le 2012-01-18]

## Description

L'invention concerne un procédé technique ainsi qu'un dispositif de maintenance d'un équipement d'un véhicule, et plus particulièrement un aéronef.

L'invention se situe donc en particulier dans le domaine technique des systèmes de maintenance embarqués, et plus spécifiquement des systèmes de maintenance pour des équipements électroniques.

Classiquement, un constructeur spécifie la durée de vie d'un équipement, puis peut effectuer des essais pour démontrer que la durée de vie de cet équipement est conforme aux exigences. Par exemple, cette durée de vie peut être évaluée en nombre d'heures de vol, 10000 heures de vol par exemple pour un giravion.

Les équipements d'aéronef subissent des vibrations non négligeables susceptibles de les détériorer. Il convient donc de vérifier que ces vibrations ne sont pas de nature à réduire la durée de vie de l'équipement. Dès lors, on réalise des essais vibratoires sur l'équipement.

On comprend toutefois qu'il est difficile de réaliser des essais durant la durée de vie complète de l'équipement, un essai de 10000 heures par exemple étant difficilement concevable à des coûts raisonnables.

Par suite, on soumet l'équipement à un spectre vibratoire plus élevé que le spectre vibratoire auquel est soumis l'équipement en utilisation, mais durant une durée inférieure à la durée de vie prévisionnelle de cet équipement.

Durant une phase de définition, un équipement peut donc être soumis à un test accéléré et destructeur en vibration représentant les sollicitations appliquées sur cet équipement pendant sa durée de vie spécifiée.

Le spectre vibratoire appliqué durant un tel essai et la durée de cet essai sont obtenus par des méthodes usuelles associant par exemple une durée à un niveau vibratoire.

Par ailleurs, un véhicule et notamment un aéronef peut être muni de systèmes antivibratoires actifs et passifs. Le spectre vibratoire auquel est soumis un équipement est donc déterminé en prenant en considération l'implémentation de tels systèmes antivibratoires.

Ces systèmes antivibratoires sont conçus pour présenter un niveau de fiabilité très élevé. Néanmoins, une panne n'est pas à exclure, bien qu'extrêmement rare.

Par suite, en cas de panne d'un système antivibratoire, un équipement peut être soumis à des vibrations hors norme. L'endommagement de cet équipement peut être sensiblement augmenté d'un facteur dix par exemple.

Or, de tels niveaux vibratoires ne sont pas toujours pris en considération durant la phase de définition de l'équipement, en raison de leur caractère improbable. Ainsi, lorsque l'augmentation du niveau vibratoire subi par l'équipement se produit sur une durée non négligeable, il en résulte une diminution du potentiel de vie de cet équipement.

On comprend qu'il se pose alors le problème de la durée de vie d'équipements soumis à des contraintes vibratoires indues, et de la maintenance prédictive de ces équipements.

On connaît des systèmes à base de « sentinelles » mesurant en temps réel les niveaux vibratoires au niveau d'équipements, notamment des cartes électroniques. Ces systèmes permettent de mesurer en temps réel les vibrations subies par un équipement et d'en déduire la durée de vie restante de cet équipement.

Ces systèmes sont très intéressants mais présentent un coût non négligeable.

Ainsi, le document EP0541277 décrit un système de vérification d'état à réduction intégrée de vibrations. Des outils de maintenance, avec des actions de maintenance d'un équipement en fonction de sa dégradation est prévu, par exemple pour un hélicoptère. Une réduction de vibrations emploie la détermination de fonctionnement sous un mode normal ou dégradé. Ce document correspond au préambule de la revendication 1.

Le document XP55016804 décrit des charges d'utilisation de cadres structurels et des usages de vérification dans un hélicoptère. Des régimes de vol sont reconnus pour ajuster la détermination d'usure effective de composants. Des durées relatives de fonctionnement de composants sont prévues, en fonction de régimes et de situations diverses de fonctionnement, auquel des taux de dommages par fatigue sont couplés.

Ces durées relatives font référence à des pourcentages d'une durée de vie.

La présente invention a alors pour objet de proposer un procédé alternatif à bas coût pour réaliser la maintenance d'un équipement.

L'invention est définie par les revendications 1 à 5 de procédé, 6 de dispositif et 7 d'aéronef. Ses avantages apparaîtront avec plus de détails dans le cadre de la description qui suit avec des exemples de réalisation donnés à titre illustratif en référence à la figure unique annexée.

Selon une réalisation de l'invention, un procédé technique de maintenance d'un équipement d'un véhicule comprenant un système antivibratoire, est notamment remarquable en ce que :
- on détermine si le système antivibratoire fonctionne selon un mode de fonctionnement normal ou selon un mode de fonctionnement dégradé,
- on détermine un premier nombre d'heures de fonctionnement de l'équipement lorsque le système antivibratoire fonctionne selon le mode de fonctionnement normal,
- on détermine un deuxième nombre d'heures de fonctionnement de l'équipement lorsque le système antivibratoire fonctionne selon le mode de fonctionnement dégradé,
- on convertit le deuxième nombre d'heures de fonctionnement en un troisième nombre d'heures de fonctionnement en appliquant une loi de conversion prédéterminée, le troisième nombre d'heures de fonctionnement générant dans les conditions du mode de fonctionnement normal un endommagement de l'équipement égal à un endommagement provoqué par un fonctionnement dudit équipement durant le deuxième nombre d'heures de fonctionnement,
- on agit sur l'équipement lorsque la somme du premier nombre et du troisième nombre atteint un seuil prédéterminé.

Il est à noter que l'on entend par système antivibratoire un moyen pour réduire les vibrations, et notamment les vibrations de la structure de l'aéronef. Dans le cadre d'un giravion, le système antivibratoire peut induire un moyen agencé sur un rotor de sustentation du giravion ou encore sur une boîte de transmission de puissance entraînant ce rotor de sustentation par exemple.

De plus, on entend par « heures de fonctionnement » le temps passé par un équipement sur un véhicule en fonctionnement, à savoir à partir du moment où un pilote agit sur le véhicule pour le démarrer jusqu'au moment où un pilote arrête ce véhicule. Ce temps n'est pas assimilable à un pourcentage d'une durée de vie.

Ainsi, selon l'invention, durant une phase de définition, un constructeur détermine un seuil prédéterminé, correspondant à une durée de vie prévisionnelle d'un équipement puis détermine un spectre vibratoire subi par cet équipement durant cette durée de vie. Ce spectre vibratoire est dénommé par commodité « spectre vibratoire estimé ».

Le constructeur vérifie la conformité de l'équipement en réalisant un essai accéléré simulant le fonctionnement de l'équipement durant sa durée de vie selon le spectre vibratoire estimé.

En utilisation, et notamment en vol dans le cadre d'un véhicule de type aéronef, on détermine alors le premier nombre d'heures de fonctionnement en conditions normales, à savoir lorsque le système antivibratoire fonctionne selon le mode de fonctionnement normal.

De même, on comptabilise la durée de fonctionnement de l'équipement en cas de dysfonctionnement du système antivibratoire. Ainsi, lorsque le système antivibratoire fonctionne selon le mode de fonctionnement dégradé, on détermine un deuxième nombre d'heures de fonctionnement dudit équipement.

Par suite, à l'aide d'une loi de conversion prédéterminée par le constructeur, on convertit ce deuxième nombre d'heures de fonctionnement en un troisième nombre d'heures de fonctionnement. Par exemple, on peut établir qu'une heure de fonctionnement durant le mode de fonctionnement dégradé est égale à dix heures de fonctionnement durant le mode de fonctionnement normal en termes d'endommagement de l'équipement.

Il est à noter que l'on entend par « loi » tout moyen fournissant une information permettant de réaliser la conversion requise, à savoir par exemple, une relation mathématique, une base de données, des diagrammes ou équivalents.

Enfin, on agit sur l'équipement lorsque la somme du premier nombre et du troisième nombre atteint un seuil prédéterminé, à savoir la durée de vie par exemple. Cette étape consiste à un acte technique réalisé sur l'équipement pour le démonter, puis le soumettre à une action de maintenance visant à prolonger son utilisation voire pour le remplacer.

Ce procédé ne nécessite donc pas l'emploi de moyens dédiés onéreux. Il répond alors au problème technique posé visant à lutter contre des effets vibratoires indus et rares sur des équipements.

L'invention peut aussi comprendre une ou plusieurs des caractéristiques qui suivent.

Par exemple, le seuil prédéterminé peut être égal à la durée de vie prédéterminée de l'équipement.

Selon un autre aspect, le système antivibratoire fonctionne selon le mode de fonctionnement dégradé lorsque ce système antivibratoire est au moins partiellement en panne.

En outre, le système antivibratoire est éventuellement un dispositif actif.

Par ailleurs, pour déterminer ladite loi de conversion :
- on estime le spectre vibratoire dudit équipement en utilisation lorsque ledit système antivibratoire fonctionne en mode dégradé et on en déduit un niveau vibratoire moyen,
- on détermine un seuil alternatif associé audit niveau vibratoire moyen,
- on en déduit une loi de conversion correspondant à un coefficient multiplicatif égal au quotient du seuil prédéterminé et du seuil alternatif.

Outre un procédé, l'invention vise un dispositif de maintenance d'un équipement d'un véhicule comprenant un système antivibratoire.

Ce dispositif est notamment remarquable en ce qu'il comporte un organe périphérique et une unité de traitement communiquant avec ledit système antivibratoire, l'unité de traitement étant munie d'une mémoire, l'unité de traitement exécutant des instructions stockées dans la mémoire afin de mettre en oeuvre le procédé explicité précédemment pour notamment:
- déterminer si le système antivibratoire fonctionne selon un mode de fonctionnement normal ou selon un mode de fonctionnement dégradé,
- déterminer un premier nombre d'heures de fonctionnement de l'équipement lorsque le système antivibratoire fonctionne selon le mode de fonctionnement normal,
- déterminer un deuxième nombre d'heures de fonctionnement de l'équipement lorsque le système antivibratoire fonctionne selon le mode de fonctionnement dégradé,

- pour convertir le deuxième nombre d'heures de fonctionnement en un troisième nombre d'heures de fonctionnement en appliquant une loi de conversion prédéterminée,
- pour signaler à un opérateur la somme du premier nombre d'heures de vol et du troisième nombre d'heures de fonctionnement.

Enfin, l'invention vise un aéronef muni de ce dispositif.

L'invention et ses avantages apparaîtront avec plus de détails dans le cadre de la description qui suit avec des exemples de réalisation donnés à titre illustratif en référence aux figures suivantes qui représentent :
- la figure 1, un schéma d'un véhicule selon l'invention, et
- la figure 2, un schéma explicitant un procédé selon l'invention.

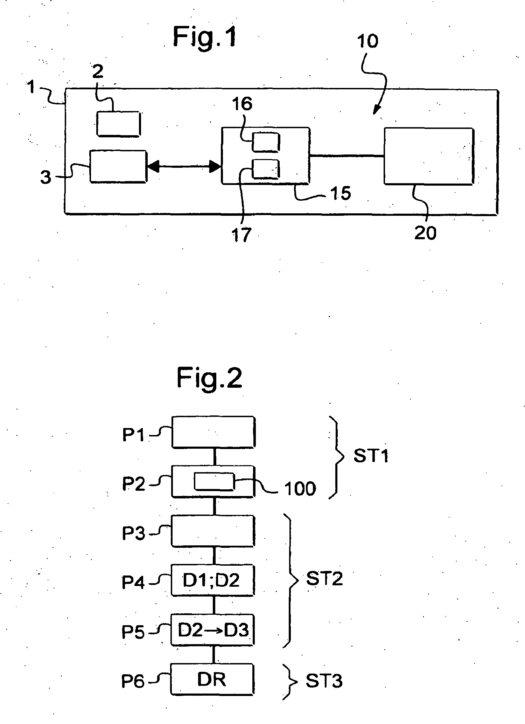

La figure 1 présente un véhicule 1 selon l'invention, un aéronef plus particulièrement.

Il est à noter que le véhicule est schématisé pour ne pas alourdir inutilement la figure 1.

Ce véhicule 1 comprend au moins un équipement 2, tel qu'une carte électronique embarquée. De plus, le véhicule 1 est muni d'un système antivibratoire pour au moins réduire les vibrations d'une structure porteuse de ce véhicule ce système antivibratoire pouvant être un dispositif actif.

En outre, le véhicule 1 est muni d'un dispositif 10 de maintenance de l'équipement 2.

Ce dispositif 10 inclut une unité de traitement 15 communiquant avec le système antivibratoire de manière à recevoir un signal discret de panne le cas échéant. De plus, le dispositif 10 comporte un organe périphérique 20 communiquant avec l'unité de traitement 15, cet organe périphérique pouvant être un écran d'affichage ou tout autre moyen de signalisation d'une information.

Dès lors, l'unité de traitement 15 possède une mémoire 16 et éventuellement au moins un processeur 17, le processeur exécutant des instructions stockées dans la mémoire 16 pour indiquer à un opérateur d'agir sur l'équipement. Par exemple, l'unité de traitement commande l'affichage d'un ordre de maintenance sur l'organe périphérique 20.

La figure 2 explicite notamment le procédé automatisé mis en oeuvre par le dispositif 10.

Durant une phase de définition ST1, au cours d'une étape de spécification P1, le constructeur du véhicule spécifie un seuil prédéterminé D0 à partir duquel un opérateur doit agir sur un équipement 2.

Par exemple, le seuil prédéterminé D0 est la durée de vie prévisionnelle que doit atteindre l'équipement 2 dans des conditions de fonctionnement normal, à savoir en dehors de pannes annexes.

Durant une étape de qualification P2, le constructeur vérifie que l'équipement 2 répond aux exigences requises, et présente par exemple la durée de vie prévisionnelle.

A l'aide de méthodes usuelles 100, le constructeur réalise un essai vibratoire accéléré en soumettant l'équipement 2 à un niveau vibratoire supérieur au niveau vibratoire que l'équipement 2 va subir lors du fonctionnement du véhicule. De plus, cet essai vibratoire est réalisé pendant une durée inférieure au seuil prédéterminé, à savoir à la durée de vie prévisionnelle.

Durant une phase de recensement ST2 réalisée à bord d'un véhicule, au cours d'une étape d'analyse P3, l'unité de traitement détermine si le système antivibratoire 3 fonctionne selon un mode de fonctionnement normal ou selon un mode de fonctionnement dégradé.

Par exemple, le système antivibratoire 3 envoie un signal discret de panne à l'unité de traitement 15 durant le mode de fonctionnement dégradé. On se référera à la littérature pour obtenir des informations sur une telle signalisation d'un mode de fonctionnement dégradé.

On note que l'on peut considérer que le système antivibratoire fonctionne dans le mode de fonctionnement dégradé lorsqu'il est en totalement ou partiellement en panne.

Au cours d'une étape de comptage P4, l'unité de traitement 15 détermine :
- un premier nombre D1 d'heures de fonctionnement de l'équipement 2 lorsque le système antivibratoire 3 fonctionne selon le mode de fonctionnement normal,
- un deuxième nombre D2 d'heures de fonctionnement de l'équipement 2 lorsque le système antivibratoire 3 fonctionne selon le mode de fonctionnement dégradé.

De plus, durant une étape de conversion P5, l'unité de traitement 15 détermine un troisième nombre D3 d'heures de fonctionnement en convertissant le deuxième nombre D2 d'heures de fonctionnement à l'aide d'une loi de conversion prédéterminée.

Il est à noter que l'utilisation de l'équipement durant le troisième nombre D3 d'heures de fonctionnement dans les conditions du mode de fonctionnement normal induit un endommagement de l'équipement 2 égal à l'endommagement provoqué par un fonctionnement de cet équipement 2 durant le deuxième nombre d'heures de fonctionnement dans les conditions du mode de fonctionnement dégradé.

Pour déterminer la loi de conversion, durant la phase de définition ST1, on peut estimer le spectre vibratoire de l'équipement 2 en utilisation lorsque le système antivibratoire 3 fonctionne en mode dégradé pour en déduire un niveau vibratoire moyen.

Dès lors, à l'aide par exemple des relations utilisées durant l'étape P2, on peut déterminer un seuil alternatif D0' associé audit niveau vibratoire moyen, et en déduire une loi de conversion correspondant à un coefficient multiplicatif égal au quotient du seuil prédéterminé D0 et du seuil alternatif D0'.

Durant une étape de commande P6 d'une phase de commande ST3, l'unité de traitement 15 détermine si la somme du premier nombre D1 d'heures de fonctionnement et du troisième nombre D3 d'heures de fonctionnement atteint un seuil prédéterminé D0 et ordonne à un opérateur d'agir sur ledit équipement 2 le cas échéant.

Par exemple, l'unité de traitement détermine une durée de vie restante DR égale à la durée de vie prévisionnelle D0 à laquelle on retranche la somme du premier nombre D1 et du deuxième nombre D3 soit : DR=D0-(D1+D3).

Si la durée de vie restante atteint zéro, un opérateur en est informé via l'organe périphérique 20. L'opérateur peut alors réviser l'équipement 2 ou encore le remplacer par exemple.

Naturellement, la présente invention est sujette à de nombreuses variations quant à sa mise en oeuvre. Bien que plusieurs modes de réalisation aient été décrits, on comprend bien qu'il n'est pas concevable d'identifier de manière exhaustive tous les modes possibles. Il est bien sûr envisageable de remplacer un moyen décrit par un moyen équivalent sans sortir du cadre de la présente invention, comme elle est définie dans les revendications.

## Revendications

1. Procédé technique de maintenance d'un équipement (2) d'un véhicule (1) comprenant un système antivibratoire (3), et les étapes suivantes :
- on détermine si ledit système antivibratoire (3) fonctionne selon un mode de fonctionnement normal ou selon un mode de fonctionnement dégradé, **caractérisé par** les étapes suivantes :
- on détermine un premier nombre (D1) d'heures de fonctionnement dudit équipement (2) lorsque ledit système antivibratoire (3) fonctionne selon le mode de fonctionnement normal,
- on détermine un deuxième nombre (D2) d'heures de fonctionnement dudit équipement (2) lorsque ledit système antivibratoire (3) fonctionne selon le mode de fonctionnement dégradé,
- on convertit ledit deuxième nombre (D2) d'heures de fonctionnement en un troisième nombre (D3) d'heures de fonctionnement en appliquant une loi de conversion prédéterminée, le troisième nombre (D3) d'heures de fonctionnement générant dans les conditions du mode de fonctionnement normal un endommagement de l'équipement (2) égal à un endommagement provoqué par un fonctionnement dudit équipement (2) durant le deuxième nombre d'heures de fonctionnement,
- on agit sur ledit équipement (2) lorsque la somme du premier nombre (D1) et du troisième nombre (D3) atteint un seuil prédéterminé (D0).

2. Procédé selon la revendication 1,
**caractérisé en ce que** ledit seuil prédéterminé (D0) est égal à la durée de vie prédéterminée dudit équipement.

3. Procédé selon l'une des revendications 1 à 2,
**caractérisé en ce que** ledit système antivibratoire (3) fonctionne selon le mode de fonctionnement dégradé lorsque ce système antivibratoire (3) est au moins partiellement en panne.

4. Procédé selon l'une des revendications 1 à 3,
**caractérisé en ce que** ledit système antivibratoire (3) est un dispositif actif.

5. Procédé selon l'une des revendications 1 à 4, **caractérisé en ce que**, pour déterminer ladite loi de conversion :
on estime le spectre vibratoire dudit équipement (2) en utilisation lorsque ledit système antivibratoire (3) fonctionne en mode dégradé et on en déduit un niveau vibratoire moyen,
on détermine un seuil alternatif (D0') associé audit niveau vibratoire moyen,
on en déduit une loi de conversion correspondant à un coefficient multiplicatif égal au quotient du seuil prédéterminé (D0) et du seuil alternatif (D0').

6. Dispositif (10) de maintenance d'un équipement (2) d'un véhicule (1) comprenant un système antivibratoire (3),
un organe périphérique (20) et une unité de traitement (15) communiquant avec ledit système antivibratoire (3), ladite unité de traitement (15) étant munie d'une mémoire (16), **caractérisé en ce que** ladite unité de traitement (15) est adaptée pour exécuter des instructions stockées dans ladite mémoire (16) afin de mettre en oeuvre le procédé selon l'une quelconque des revendications 1 à 5:
- pour déterminer si ledit système antivibratoire fonctionne selon un mode de fonctionnement normal ou selon un mode de fonctionnement dégradé,
- pour déterminer un premier nombre d'heures de fonctionnement dudit équipement lorsque ledit système antivibratoire fonctionne selon le mode de fonctionnement normal,
- pour déterminer un deuxième nombre d'heures de fonctionnement dudit équipement lorsque ledit système antivibratoire fonctionne selon le mode de fonctionnement dégradé,
- pour convertir ledit deuxième nombre d'heures de fonctionnement en un troisième nombre d'heures de fonctionnement en appliquant une loi de conversion prédéterminée,
- pour signaler à un opérateur la somme du premier nombre d'heures de vol et du troisième nombre d'heures de fonctionnement.

7. Aéronef (1) muni d'un équipement (2) et d'un système antivibratoire (3),
**caractérisé en ce qu'**il comporte un dispositif (10) selon la revendication 6.

## Patentansprüche

1. Technisches Verfahren zur Wartung einer Ausrüstung (2) eines Fahrzeugs (1), welches ein Antivibrationssystem (3) aufweist, mit den folgenden Schritten:
- man bestimmt, ob das Antivibrationssystem (3) in einem normalen Betriebsmodus oder in einem verschlechterten Betriebsmodus arbeitet,
**gekennzeichnet, durch** die folgenden Schritte:
- man bestimmt eine erste Anzahl (D 1) von Betriebsstunden der Ausrüstung (2), wenn das Antivibrationssystem (3) in einem normalen Betriebsmodus arbeitet,
- man bestimmt eine zweite Anzahl (D2) von Betriebsstunden der Ausrüstung (2), wenn das Antivibrationssystem (3) in dem verschlechterten Betriebsmodus arbeitet,
- man wandelt die zweite Anzahl (D2) von Betriebsstunden in eine dritte Anzahl (D3) von Betriebsstunden um **durch** Anwendung einer vorbestimmten Umwandlungsregel, wobei die dritte Anzahl (D3) von Betriebsstunden unter den Bedingungen des normalen Betriebsmodus eine Beschädigung der Ausrüstung erzeugt, die gleich einer Beschädigung ist, die **durch** einen Betrieb der Ausrüstung (2) während der zweiten Anzahl von Betriebsstunden hervorgerufen wird,
- man wirkt auf die Ausrüstung (2) ein, wenn die Summe der ersten Anzahl (D1) und der dritten Anzahl (D3) einen vorbestimmten Schwellenwert (D0) erreicht.

2. Verfahren nach Anspruch 1,
**dadurch gekennzeichnet, dass** der vorbestimmte Schwellenwert (D0) gleich der vorbestimmten Lebensdauer der Ausrüstung ist.

3. Verfahren nach einem der Ansprüche 1 bis 2,
**dadurch gekennzeichnet, dass** das Antivibrationssystem (3) in einem verschlechterten Betriebsmodus arbeitet, wenn das Antivibrationssystem (3) zumindest teilweise defekt ist.

4. Verfahren nach einem der Ansprüche 1 bis 3,
**dadurch gekennzeichnet, dass** das Antivibrationssystem (3) eine aktive Vorrichtung ist.

5. Verfahren nach einem der Ansprüche 1 bis 4,
**dadurch gekennzeichnet, dass**, um die Umwandlungsregel zu bestimmen:
das Schwingungsspektrum der verwendeten Ausrüstung (2) abgeschätzt wird, wenn das Antivibrationssystem (3) im verschlechterten Modus arbeitet, und ein mittleres Schwingungsniveau abgeleitet wird,
ein alternativer Schwellenwert (D0') bestimmt wird, der mit dem mittleren Schwingungsniveau verbunden ist,
eine Umwandlungsregel abgleitet wird, die einem Multiplikationskoeffizienten entspricht, der gleich dem Quotienten aus dem vorbestimmten Schwellenwert (D0) und dem alternativen Schwellenwert (D0') ist.

6. Vorrichtung (10) zur Wartung einer Ausrüstung (2) eines Fahrzeugs (1), welches ein Antivibrationssystem (3) aufweist, wobei ein Randelement (20) und eine Verarbeitungseinheit (15) mit dem Antivibrationssystem (3) kommunizieren, wobei die Verarbeitungseinheit (15) mit einem Speicher (16) versehen ist,
**dadurch gekennzeichnet, dass** die Verarbeitungseinheit (15) ausgebildet ist zur Ausführung von in dem Speicher (16) gespeicherten Befehlen, um das Verfahren nach einem der Ansprüche 1 bis 5 durchzuführen:
- um zu bestimmen, ob das Antivibrationssystem in einem normalen Betriebsmodus oder in einem verschlechterten Betriebsmodus arbeitet,
- um eine erste Anzahl von Betriebsstunden der Ausrüstung zu bestimmen, wenn das Antivibrationssystem in dem normalen Betriebsmodus arbeitet,
- um eine zweite Anzahl von Betriebsstunden der Ausrüstung zu bestimmen, wenn das Antivibrationssystem in dem verschlechterten Betriebsmodus arbeitet,
- um die zweite Anzahl von Betriebsstunden in eine dritte Anzahl von Betriebsstunden umzuwandeln durch Anwendung einer vorbestimmten Umwandlungsregel,
- um einer Bedienungsperson die Summe aus der ersten Anzahl von Flugstunden und der dritten Anzahl von Betriebsstunden zu melden.

7. Luftfahrzeug (1) mit einer Ausrüstung (2) und einem Antivibrationssystem (3),
**dadurch gekennzeichnet, dass** es eine Vorrichtung (10) nach Anspruch 6 aufweist.

## Claims

1. Technical method for maintenance of a piece of equipment (2) of a vehicle (1) comprising an anti-vibration system (3), and the following steps:
- it is determined whether said anti-vibration system (3) is operating in a normal mode of operation or in a degraded mode of operation, **characterised by** the following steps:
- a first number (D1) of hours of operation of said piece of equipment (2) when said anti-vibration system (3) is operating in the normal mode of operation is determined,
- a second number (D2) of hours of operation of said piece of equipment (2) when said anti-vibration system (3) is operating in the degraded mode of operation is determined,
- said second number (D2) of hours of operation is converted into a third number (D3) of hours of operation by applying a predetermined conversion relationship, the third number (D3) of hours of operation generating under normal mode of operation conditions damage to the piece of equipment (2) equal to damage caused by operation of said piece of equipment (2) during the second number of hours of operation,
- said piece of equipment (2) is acted on when the sum of the first number (D1) and the third number (D3) reaches a predetermined threshold (D0).

2. Method according to claim 1,
**characterised in that** said predetermined threshold (D0) is equal to the predetermined lifetime of said piece of equipment.

3. Method according to one of claims 1 to 2,
**characterised in that** said anti-vibration system (3) operates in the degraded mode of operation when said anti-vibration system (3) is at least partially inoperative.

4. Method according to one of claims 1 to 3,
**characterised in that** said anti-vibration system (3) is an active device.

5. Method according to one of claims 1 to 4, **characterised in that**, in order to determine said conversion relationship:
the vibratory spectrum of said piece of equipment (2) in use when said anti-vibration system (3) is operating in degraded mode is estimated and a mean vibration level is deduced therefrom,
an alternative threshold (DO') associated with said mean vibration level is determined,
a conversion relationship corresponding to a multiplicative coefficient equal to the quotient of the predetermined threshold (D0) and the alternative threshold (D0') is deduced therefrom.

6. Device (10) for maintenance of a piece of equipment (2) of a vehicle (1) comprising an anti-vibration system (3),
a peripheral member (20) and a processing unit (15) communicating with said anti-vibration system (3), said processing unit (15) being provided with a memory (16), **characterised in that** said processing unit (15) is adapted to execute instructions stored in said memory (16) in order to carry out the method according to any one of claims 1 to 5:
- to determine whether said anti-vibration system is operating in a normal mode of operation or in a degraded mode of operation,
- to determine a first number of hours of operation of said piece of equipment when said anti-vibration system is operating in the normal mode of operation,
- to determine a second number of hours of operation of said piece of equipment when said anti-vibration system is operating in the degraded mode of operation,
- to convert said second number of hours of operation into a third number of hours of operation by applying a predetermined conversion relationship,
- to signal to an operator the sum of the first number of hours of flight and the third number of hours of operation.

7. Aircraft (1) provided with a piece of equipment (2) and an anti-vibration system (3),
**characterised in that** it comprises a device (10) according to claim 6.
